Europäisches Patentamt

European Patent Office

Office européen des brevets

(11) Veröffentlichungsnummer: **0 278 096**
**A2**

(12) # EUROPÄISCHE PATENTANMELDUNG

(21) Anmeldenummer: 87118733.2

(22) Anmeldetag: 17.12.87

(51) Int. Cl.⁴: **G01N 33/00**

(30) Priorität: 13.02.87 DE 3704533

(43) Veröffentlichungstag der Anmeldung:
17.08.88 Patentblatt 88/33

(84) Benannte Vertragsstaaten:
CH ES FR GB IT LI SE

(71) Anmelder: Kernforschungszentrum Karlsruhe
GmbH
Weberstrasse 5 Postfach 3640
D-7500 Karlsruhe 1(DE)

(72) Erfinder: Braun, Hartmut, Dr.
Lebrechtstrasse 45d
D-7500 Karlsruhe(DE)
Erfinder: Metzger, Michael
Eulenweg 2
D-7504 Weingarten(DE)

(54) Verfahren zur kontinuierlichen Überwachung von Emissionen und Immissionen auf Quecksilber.

(57) Die Erfindung betrifft ein Verfahren zur kontinuierlichen Überwachung von Emissionen und Immissionen in bezug auf Quecksilber, wobei Proben aus Abgasen entnommen und auf ihren Hg-Anteil unabhängig von der Erscheinungsform quantitativ bestimmt werden.

Die der Erfindung gestellte Aufgabe besteht darin, das e.g. Verfahren derart auszubilden, daß das Hg kontinuierlich sowohl emissions-als auch immissionsseitig überwacht werden kann.

Die Aufgabe wird dadurch gelöst, daß

    a) die aus den Abgasen entommenen Teilströme durch eine Konverteranlage geleitet werden, in der das Quecksilber in atomare Form gebracht wird, und daß

    b) die aus der Konverteranlage wieder austretenden Abgase einer Meßeinrichtung zugeführt werden, in der die Konzentration des atomaren Quecksilbers mittels chemischer und/oder physikalischer Methoden nachgewiesen wird.

EP 0 278 096 A2

## Verfahren zur kontinuierlichen Überwachung von Emissionen und Immissionen auf Quecksilber

Die Erfindung betrifft ein Verfahren zur kontinuierlichen Überwachung von Emissionen und Immissionen in bezug auf Quecksilber, wobei Proben aus Abgasen entnommen und auf ihren Hg-Anteil unabhängig von der Erscheinungsform quantitativ bestimmt werden.

Bei der Verbrennung quecksilberhaltiger Brennstoffe wird Quecksilber aufgrund seiner hohen Flüchtigkeit praktisch vollständig in das Abgas freigesetzt. Quecksilber wird dabei durch vorhandenes Chlorwasserstoffgas quantitativ zu Quecksilber-(II)-chlorid oxidiert und dann aus dem Kamin emittiert, falls keine geeigneten Abgasreinigungsverfahren vorhanden sind. Wird das Abgas durch Naßwäscher gereinigt, so kann neben der nicht quantitativ abgeschiedenen Hg(II)-Komponente auch noch elementares Hg, gebildet durch Wechselwirkungen mit dem Abgas, aus der Anlage emittiert werden.

Die in der TA-Luft vorgeschriebenen Emissionsbegrenzungen für Quecksilber von 200 $\mu$g/Nm$^3$ können nur von wenigen der in Betrieb befindlichen Abfallverbrennungsanlagen eingehalten werden. Wegen der hohen Toxizität der verschiedenen Quecksilberformen wurde für die immissionsseitige Exposition ein MAK-Wert von 100 $\mu$g/Nm$^3$ (Hg-Metall und anorganische Hg-Verbindungen) festgelegt. Zudem muß eine zuverlässige Überwachung der Emissions-und Immissionsgrenzwerte unabhängig von der Quecksilberform durchführbar sein.

Eine kontinuierliche Überwachung der festgelegten Emissions-und Immissionsgrenzwerte ist im Bezug auf Quecksilber derzeit nicht möglich. Die Überwachung der Grenzwerte erfolgt diskontinuierlich nach folgenden 2 Verfahrensweisen:

### 1. Absorption in Lösungen

Unabhängig von seiner Erscheinungsform läßt sich Quecksilber aus dem Abgas quantitativ in stark sauren oxidierenden Waschlösungen absorbieren. Ein Teilstrom des quecksilberhaltigen Abgases wird hierzu durch zwei hintereinander angeordnete Impinger, gefüllt mit salpetersaurer Peroxodisulfatlösung oder schwefelsaurer Permanganatlösung, geleitet. Nach Analyse des Quecksilbergehaltes in der Absorptionslösung wird unter Berücksichtigung des Abgasvolumens die entsprechende Quecksilberemission berechnet.

### 2. Adsorption an Feststoffen

Für die Erfassung von elementarem und anorganisch gebundenem Quecksilber stehen verschiedene Feststoffadsorber zur Verfügung. Besonders geeignet ist dabei Jodkohle. Ein Teilstrom des quecksilberhaltigen Abgases wird hierzu durch einen beheizten Jodkohleadsorber geleitet und der Adsorber dann chemisch aufgeschlossen, um aus der adsorbierten Quecksilbermenge bei bekanntem Abgasvolumen die entsprechend Quecksilberemission berechnen zu können.

Diese diskontinuierlichem Meßverfahren sind nur mit erheblichem Aufwand durchführbar. Analytische Informationen können nicht am Ort der Probenahme erhalten werden. Die inputseitige und damit auch die im Abgas enthaltene Quecksilbermenge unterliegt starken Schwankungen. Diskontinuierliche Messungen, wie sie zeitweise durch die zuständigen Überwachungsstellen durchgeführt werden, führen nur zu Aussagen über Emissionszustände während der Probenahme. Eine befriedigende Überwachung der tatsächlichen Emissionssituation ist nicht möglich. Ge sundheitsgefährdungen für Personen, die durch den Umgang mit Quecksilber exponiert sind, können über diskontinuierliche Überwachung nicht mit ausreichender Sicherheit ausgeschlossen werden.

Die der Erfindung gestellte Aufgabe besteht darin, das e.g. Verfahren derart auszubilden, daß das Hg kontinuierlich sowohl emissions-als auch immissionsseitig überwacht werden kann.

Die Lösung ist in den kennzeichnenden Merkmalen des Anspruches 1 beschrieben.

Die übrigen Ansprüche geben vorteilhafte Weiterbildungen des Verfahrens an.

Erfindungsgemäß wurde daher ein geeignetes Konvertersystem entwickelt, mit welchem die primär auftretenden Quecksilber(II)-halogenide kontinuierlich in die detektierbare atomare Quecksilberform überführt werden können. Durch Kombination dieses Konverters mit z.B. einem gängigen Atomabsorptionsspektrometer ist dann die Möglichkeit einer kontinuierlichen überwachung in den verschiedenen Anwendungsbereichen geschaffen.

Die vorliegende Erfindung macht experimentell überprüfte Vorschläge zur kontinuierlichen Umwandlung der bei Emissionen und Immissionen auftretenden gasförmigen Quecksilber(II)-halogenide in die meßbare atomare Quecksilberform. Die Umwandlung kann entweder durch eine chemische Reduktion mit Kohlenstoff oder durch direkte thermische Zersetzung erfolgen.

Die in Abgas oder Umgebungsluft vorhandenen Quecksilber(II)-halogenide werden beim Hindurchleiten durch z.B. temperierten Kohlenstoff, beispielsweise in Form von Aktivkohle, kohlenstoffhaltigen Feststoffen oder eines Kalk-Kohlenstoffgemisches, vollständig in die atomare Form reduziert.

Die optimalen Umwandlungsbedingungen werden bei Reaktionstemperaturen von 330 °C erreicht. Bei dieser Temperatur ist das Verhältnis von Quecksilberdesorption und Kohlenstoffverflüchtigung besonders günstig. Große Quecksilbermengen, wie sie im Rohgas von Verbrennungsanlagen vorliegen können, lassen sich bereits bei Verweilzeiten on 0,04 s durch Kohlenstoff vollständig in die atomare Form überführen. Die in Reingasen üblichen Chlorwasserstoffkonzentrationen von 250 mg/Nm³ haben so gut wie keinen Einfluß auf die Umwandlungsreaktion. Für Rohgasmessungen, bei denen HCl-Konzentrationen von mehr als 1000 mg/Nm³ auftreten, muß zur HCl-Abtrennung ein geeigneter Absorber eingesetzt werden. Hierzu kann beispielsweise die Kohle mit einer entsprechenden Kalkmenge versetzt und dieses Gemisch gleichzeitig als HCl-Absorber und Hg-Umwandler eingesetzt werden. Mit dem Kohlenstoffkonverter lassen sich auch die in Umgebungsluft vorhandenen geringen Quecksilbergehalte zuverlässig in die atomare Form überführen.

Die Erfindung wird im folgenden anhand zweier Ausführungsbeispiele für die Umwandlungsreaktion des Hg gem. Fig. 1 und 2 sowie anhand von Experimentbeispielen 1 bis 10 mit entsprechend in Fig. 3 bis 9 dargestellten Ergebnissen beschrieben.

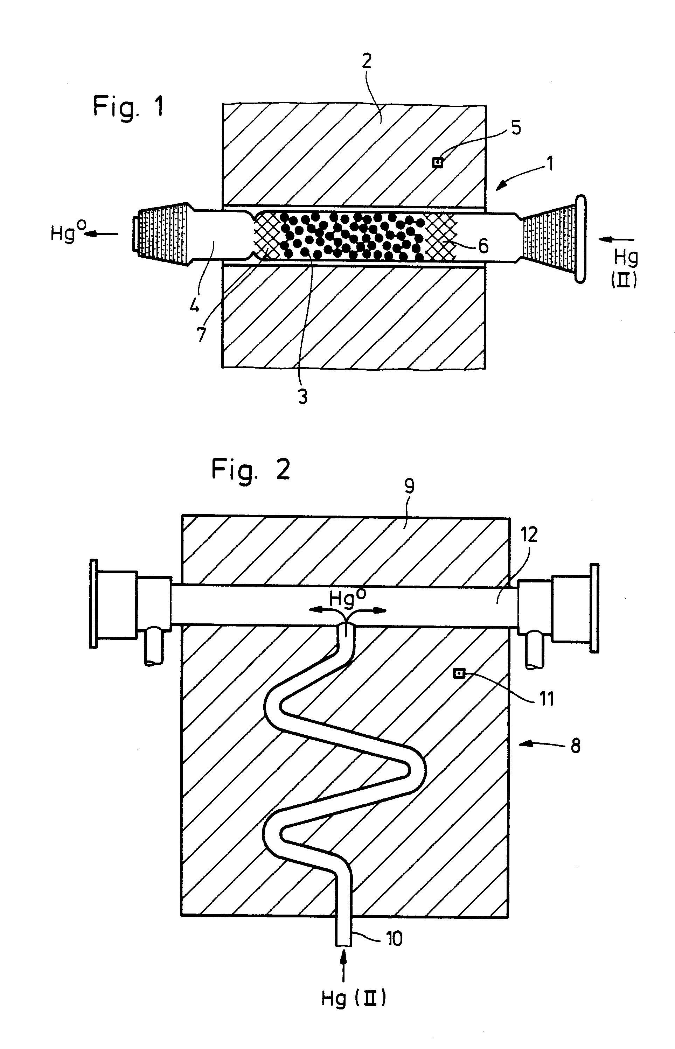

Eine kontinuierliche Umwandlung von Quecksilber(II)-Verbindungen in die atomare Quecksilberform kann mit einer Konvertereinheit gemäß Fig. 1 erfolgen. Die Konvertereinheit 1 besteht aus einem Heizmantel 2 und einem mit Kohlenstoff 3 gefüllten Reaktionsrohr 4. Der Heizmantel 2 wird elektrisch beheizt und hat einen Klappdeckel, so daß das Reaktionsrohr 4 bequem herausgenommen bzw. eingesetzt werden kann. Die Heizwendeln des Heizmantels 2 können in keramischen Mehrlochkörpern eingelegt sein, deren Kanäle zum Nutzraum hin offen sind. Zum Schutz gegen eventuelle Berührung des Heizleiters wird beim Aufklappen des Heizmantels 2 die Stromaufnahme selbständig unterbrochen und beim Schließen wieder zugeschaltet. Die Temperatur wird durch einen Sensor 5 im Heizmantel 2 überwacht und über ein Regelungssystem auf dem vorgegebenen Wert gehalten. Die Reaktionstemperatur ist im Bereich 300 bis 400 °C stufenlos einstellbar. Das Temperaturprofil des Heizmantels ist im Bereich der Kohle 3 konstant.

Das Reaktionsrohr 4 kann aus Duran-Glas gefertigt sein. Um einen möglichst geringen Strömungswiderstand im Reaktionsrohr 4 zu erreichen, sollte der zur Reduktion eingesetzte Kohlenstoff 3 in grobkörniger Form (Durchmesser 1 bis 3 mm) vorliegen. Der Kohlenstoff 3 wird durch zwei Glaswollepfropfen 6 und 7 im Glasrohr so fixiert, daß er sich in der temperaturkonstanten Zone des Heizmantels 2 befindet. Der Abstand von der Konvertereinheit 1 zum Detektionssystem (nicht dargestellt) sollte möglichst kurz gehalten werden, um Kondensationseffekte auszuschließen.

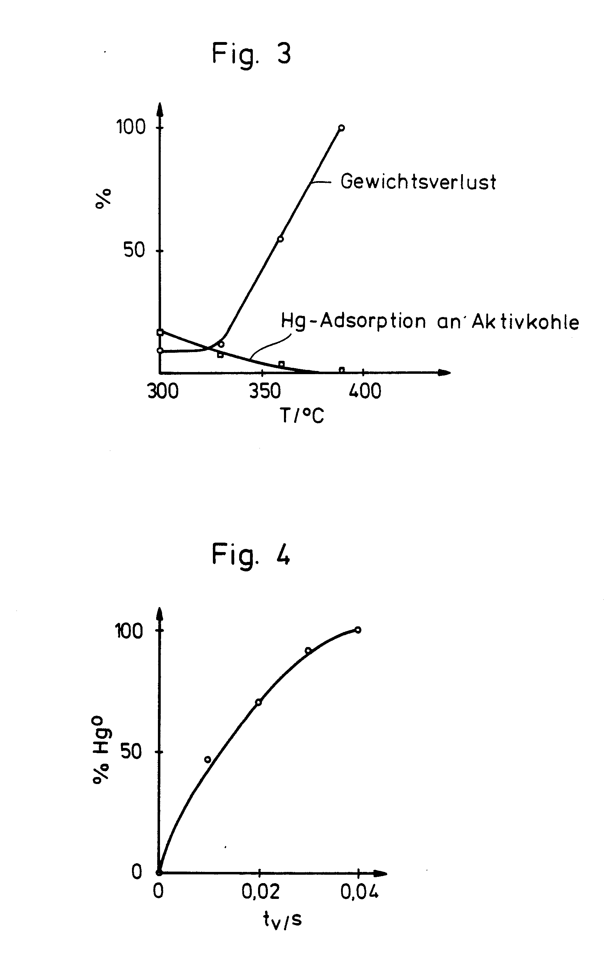

Die thermische Zersetzung kann in einer Konvertereinheit 8 gemäß Fig. 2, bestehend aus einem elektrisch beheizten Mantel 9 und einem Reaktionsrohr 10, erfolgen. Der Heizmantel 9 kann aus einem keramischen Trägerrohr, auf das der Heizleiter als Außenwicklung aufgelegt und mit keramischer Masse eingebettet ist, bestehen. Die Temperatur wird durch einen Sensor 11 im Heizmantel 9 überwacht und mit einer Regeleinheit auf den vorgegebenen Wert eingestellt. Die Arbeitstemperatur läßt sich im Bereich 700 bis 900 °C stufenlos einstellen. Die thermische Zersetzung kann in dem aus Quarzglas gefertigten Reaktionsrohr 10 erfolgen. Um bei möglichst kleiner Dimensionierung die erforderlichen Verweilzeiten zu erreichen, wird das Reaktionsrohr 10 spiralförmig angeordnet und mündet vorzugsweise innerhalb der Konvertereinheit direkt mittig in der Absorptionszelle 12 eines Spektrophotometers.

Prinzipiell läßt sich die Konvertereinheit 8 so gestalten, daß sowohl eine Kohlenstoff-Reduktion als auch eine thermische Zersetzung im gleichen Heizmantel ausgeführt werden kann. Das Konvertersystem besteht hierbei in ähnlicher Weise, wie im ersten Beispiel nach Fig. 1 beschrieben, aus einem aufklappbaren Heizmantel. Die Arbeitstemperatur ist im Bereich 300 bis 900 °C stufenlos einstellbar. Die Dimensionierung des Heizmantels muß so ausgelegt sein, daß für beide Umwandlungsarten die erforderlichen Verweilzeiten erreicht werden.

Stellvertretend für alle Quecksilber(II)-Halogenide wurde die Umwandlungsreaktion im Bezug auf Quecksilber(II)-Chlorid betrachtet. Zur Reduktion wurde Kohlenstoff in Form von gekörnter Aktivkohle AK (Ø 1-3 mm) eingesetzt. Die Identifizierung der Quecksilberformen wurde mit der Adsorberkombination Dowex (Hg(II))-Jodkohle ($Hg^0$) vorgenommen.

3

**Beispiel 1:**

Jeweils 10,0 g Aktivkohle wurde für die Dauer von 24 h bei unterschiedlichen Temperaturen erhitzt und anschließend durch Wägung der prozentuale Massenverlust bestimmt. Die Versuchsergebnisse sind in Fig. 3 dargestellt.

**Beispiel 2:**

Durch ein mit Aktivkohle gefülltes Glasrohr wurde bei unterschiedlichen Temperaturen ein Reingasstrom mit einem Quecksilbergehalt von 100 $\mu$g/Nm$^3$ geleitet. Der Reingasstrom entsprach in seiner Zusammensetzung dem einer Verbrennungsanlage ($SO_2$ = 200 mg/Nm$^3$, $NO_x$ = 200 mg/Nm$^3$, CO = 100 mg/Nm$^3$, HCl = 100 mg/Nm$^3$, O = 11 %).
Die Versuchsergebnisse sind in Fig. 3 dargestellt.

**Beispiel 3:**

Bei einer Temperatur von 350 °C wurde ein Reingasstrom mit 1000 $\mu$g Hg/Nm$^3$ mit unterschiedlichen Verweilzeiten durch Aktivkohle geleitet.
Die Versuchsergebnisse sind in Fig. 4 dargestellt.

**Beispiel 4:**

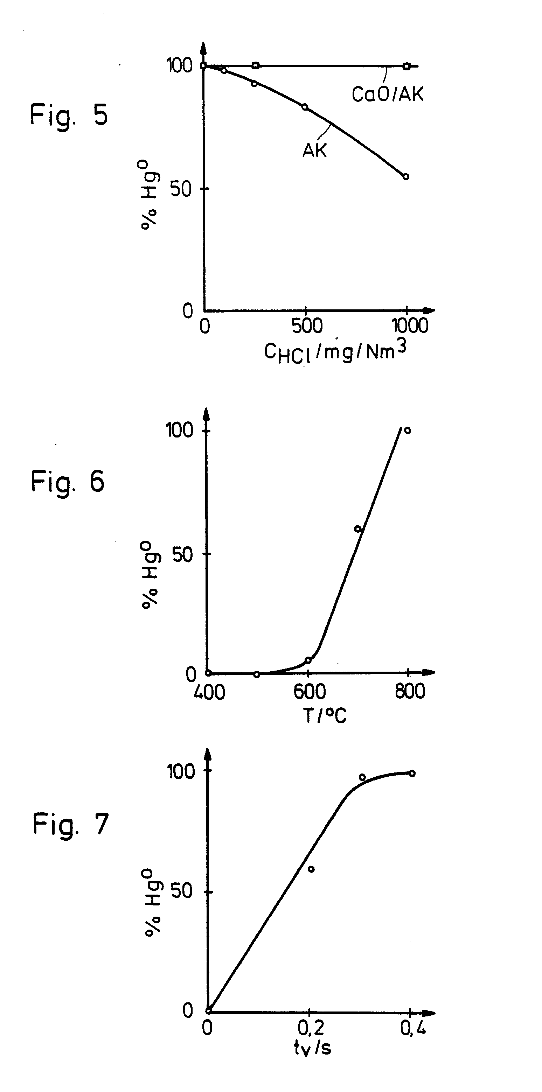

Bei einer Temperatur von 350 °C wurde ein Reingasstrom mit 100 $\mu$g Hg/Nm$^3$ bei unterschiedlichen HCl-Konzentrationen durch Aktivkohle geleitet.
Die Versuchsergebnisse sind in Fig. 5 dargestellt.

**Beispiel 5:**

Bei einer Temperatur von 350 °C wurde ein Reingasstrom mit 100 $\mu$g Hg/Nm$^3$ bei unterschiedlichen HCl-Konzentrationen durch ein Gemisch aus Aktivkohle und Calciumoxid geleitet.
Die Versuchsergebnisse sind in Fig. 5 dargestellt.

**Beispiel 6:**

Bei einer Temperatur von 350 °C wurde Umgebungsluft mit unterschiedlichen Quecksilbergehalten durch Aktivkohle geleitet.

| $c_{Hg}$ / $\mu$g/Nm$^3$ | $c_{Hg}$ gefunden / % | |
|---|---|---|
| | Hg° | Hg(II) |
| 1 | 100 | 0 |
| 10 | 100 | 0 |
| 100 | 100 | 0 |

Die Experimentbeispiele 7 bis 10 sind mit der thermischen Zersetzung mittels der Konverteranlage gemäß Fig. 2 ausgeführt worden.

Die in Abgas oder Umgebungsluft vorhandenen Quecksilber(II)-halogenide werden beim Hindurchleiten durch die ausreichend beheizte Temperaturzone vollständig unter Bildung von atomarem Quecksilber zersetzt.

Die thermische Zersetzung der Quecksilber(II)-halogenide ist bereits bei Temperaturen um 600 °C merklich. Eine vollständige Zersetzung findet bei Reaktionstemperaturen von 800 °C (Beispiel 7) bereits bei Gasverweilzeiten von 0,4 s (Beispiel 8) statt. Die zur vollständigen Umwandlung erforderlichen Verweilzeiten nehmen mit zunehmender Temperatur ab. Die Anwesenheit von Chlorwasserstoffgas wirkt der Zersetzungsreaktion entgegen. Bereits in Gegenwart von 100 mg HCl/Nm$^3$ ist das Bildungsgleichgewicht wieder weitgehend auf die Seite von Hg(II) verschoben (Beispiel 9).

Eine thermische Zersetzung ist nur dann ohne Bedenken anwendbar, wenn das Vorhandensein von Chlorwasserstoff weitgehend ausgeschlossen werden kann. Dies ist in der Regel bei Imissionsüberwachungen der Fall. Muß dagegen von der Gegenwart von HCl ausgegangen werden, so ist eine Entfernung dieser Komponente unbedingt erforderlich. Dies ist beispielsweise durch das Vorschalten eines temperierten HCl-Absorbers, beispielsweise Calciumoxid oder Silicagel, möglich (Beispiel 9). Allerdings muß die Absorbereffizienz z.B. mit Bromphenolblau, einem Reagenz auf HCl, ständig überprüft werden. Eine direkte thermische Zersetzung in der Absorptionszelle unter gleichzeitiger atomspektrometrischer Detektion ist unter Einhaltung ausreichender Verweilzeiten und Chlorwasserstoff-Ausschluß möglich (Beispiel 10).

Beispiel 7:

Ein Reingasstrom (ohne HCl) mit 1000 µg Hg/Nm$^3$ wurde durch eine unterschiedlich beheizte Temperaturzone geleitet.
Die Versuchsergebnisse sind in Fig. 6 dargestellt.

Beispiel 8:

Ein Reingasstrom (ohne HCl) mit 1000 µg Hg/Nm$^3$ wurde mit unterschiedlichen Verweilzeiten durch eine auf 800 °C beheizte Temperaturzone geleitet.
Die Versuchsergebnisse sind in Fig. 7 dargestellt.

Beispiel 9:

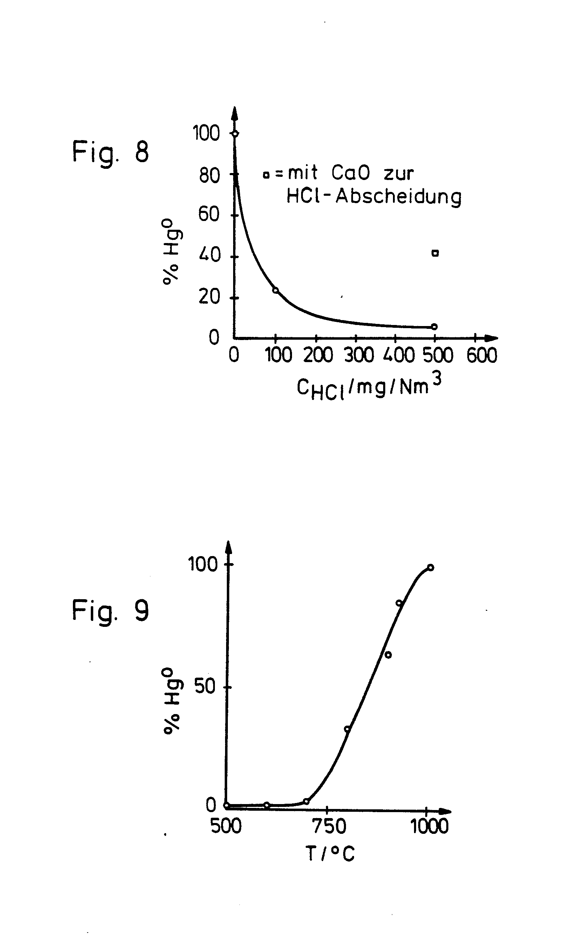

Ein Reingasstrom mit 1000 µg Hg/Nm$^3$ wurde bei unterschiedlichen HCl-Gehalten durch eine auf 800 °C beheizte Temperaturzone ohne bzw. mit vorgeschaltetem CaO-Absorber geleitet.
Die Versuchsergebnisse sind in Fig. 8 dargestellt.

Beispiel 10:

Ein Reingasstrom (ohne HCl) mit 1000 µg Hg/Nm$^3$ wurde mit einer Verweilzeit von 0,1 s durch eine unterschiedlich beheizte Absorptionszelle eines Spektrophotometers (AAS) geleitet.
Die Versuchsergebnisse sind in Fig. 9 dargestellt.

**Ansprüche**

1. Verfahren zur kontinuierlichen Überwachung von Emissionen und Immissionen in bezug auf Quecksilber, wobei Proben aus Abgasen entnommen und auf ihren Hg-Anteil unabhängig von der Erscheinungsform quantitativ bestimmt werden, dadurch gekennzeichnet, daß

a) die aus den Abgasen entnommenen Teilströme durch eine Konverteranlage (1 bzw. 8) geleitet werden, in der das Quecksilber in atomare Form gebracht wird, und daß

b) die aus der Konverteranlage (1 bzw. 8) wieder austretenden Abgase einer Meßeinrichtung zugeführt werden, in der die Konzentration des atomaren Quecksilbers mittels chemischer und/oder physikalischer Methoden nachgewiesen wird.

2. Verfahren nach Anspruch 1, dadurch gekennzeichnet, daß die Abgase durch einen temperierfähigen Feststoff und/oder ein Temperaturfeld (9, 10) hundurchgeleitet werden.

3. Verfahren nach Anspruch 1 und 2, gekennzeichnet durch die Verwendung der Atomabsorptionsspektrometrie als Nachweis für das atomare Hg.

5

4. Verfahren nach Anspruch 1 oder einem der folgenden, dadurch gekennzeichnet, daß als Feststoffbett (3) Kohle, insbesondere Aktivkohle, verwendet wird und das Feststoffbett (3) auf Temperaturen um 350 °C gehalten wird.

5. Verfahren nach Anspruch 1 oder einem der folgenden, ausgenommen Anspruch 4, dadurch gekennzeichnet, daß das Probengas durch eine Heizofenzone (9, 10) mit Temperaturen über 700 °C geführt wird.

6. Verfahren nach Anspruch 1 oder einem der folgenden, dadurch gekennzeichnet, daß in den Probengasstrom (10) oder dem Feststoffbett (3) eine Ca-haltige Substanz beigefügt wird.

Fig. 1

Fig. 2

## Fig. 3

Gewichtsverlust

Hg-Adsorption an Aktivkohle

## Fig. 4

Fig. 5

Fig. 6

Fig. 7

Fig. 8

% Hg⁰

□ = mit CaO zur
HCl-Abscheidung

$C_{HCl}/mg/Nm^3$

Fig. 9

% Hg⁰

T/°C